# EUROPEAN PATENT APPLICATION

(11) **EP 4 293 106 A1**
(43) Date of publication of application: **20.12.2023**
(21) Application number: 22752880.9
(22) Date of filing: 15.02.2022
(51) Int. Cl.: C12N 5/07, C12N 5/0775

(54) **METHOD FOR PRODUCING THREE-DIMENSIONAL TISSUE BODY AND METHOD FOR PROMOTING DIFFERENTIATION OF ADIPOSE-DERIVED STEM CELLS**

(30) Priority: 15.02.2021 JP 2021021791
(71) Applicant: TOPPAN INC., Tokyo 110-0016 (JP); OSAKA UNIVERSITY, Suita-shi Osaka 565-0871 (JP); Kyoto Prefectural Public University Corporation, Kyoto-shi, Kyoto 602-8566 (JP)
(72) Inventor: KITANO Shiro, Tokyo 110-0016 (JP); MATSUSAKI Michiya, Suita-shi, Osaka 565-0871 (JP); LOUIS Fiona, Suita-shi, Osaka 565-0871 (JP); SOWA Yoshihiro, Kyoto-shi, Kyoto 602-8566 (JP)
(74) Representative: TBK
(86) International application number: PCT/JP2022/005969
(87) International publication number: WO 2022/173058

(57) **Abstract**

A method for producing a three-dimensional tissue construct comprising mature adipocytes, comprising incubating cells comprising at least fat-derived stem cells in the presence of one or more fatty acids selected from the group consisting of erucic acid, elaidic acid, oleic acid, palmitoleic acid, myristoleic acid, phytanic acid, and pristanic acid.

## Description

### Technical Field

The present invention relates to a method for producing a three-dimensional tissue construct, and a method for promoting the differentiation of fat-derived stem cells.

### Background Art

Known as techniques to artificially produce constructs simulating biological tissues are, for example, a method of producing a three-dimensional tissue construct, the method including three-dimensionally disposing cells each coated with a coating film containing collagen to form a three-dimensional tissue construct (Patent Literature 1), and a method of producing a three-dimensional cellular tissue, the method including: mixing cells with a cationic substance and an extracellular matrix component to give a mixture; collecting cells from the resulting mixture; and forming a cell aggregate on a substrate (Patent Literature 2). The present inventors have proposed a method for producing a large-sized three-dimensional tissue construct having a thickness of 1 mm or more with use of a relatively small number of cells by bringing cells and fragmented exogenous collagen into contact with each other (Patent Literature 3).

### Citation List

### Patent Literature

Patent Literature 1: International Publication No. WO 2015/072164
Patent Literature 2: International Publication No. WO 2017/146124
Patent Literature 3: International Publication No. WO 2018/143286

### Summary of Invention

### Technical Problem

Three-dimensional tissue constructs, which are aggregates of cells artificially produced by cell culture, can be obtained according to any of the above-mentioned production methods. In particular, three-dimensional tissue constructs comprising adipocytes are expected to be used as alternatives for experimental animals, materials for transplantation, and so on, for example, and a method for promoting the differentiation of fat-derived stem cells is demanded for producing three-dimensional tissue constructs comprising adipocytes.

The present invention was made in view of the above circumstance, and an object of the present invention is to provide a method for promoting differentiation of fat-derived stem cells into mature adipocytes in producing a three-dimensional tissue construct comprising mature adipocytes.

### Solution to Problem

The present inventors diligently studied to find that the differentiation of fat-derived stem cells into mature adipocytes is promoted by incubating fat-derived stem cells in the presence of a TGFβ type I receptor inhibitor and/or a specific fatty acid, which led to the completion of the present invention.

Specifically, the present invention includes, for example, the followings.
[1] A method for producing a three-dimensional tissue construct comprising mature adipocytes, comprising incubating cells comprising at least fat-derived stem cells in the presence of one or more fatty acids selected from the group consisting of erucic acid, elaidic acid, oleic acid, palmitoleic acid, myristoleic acid, phytanic acid, and pristanic acid.
[2] The method of [1], comprising incubating the cells in the presence of a TGFβ type I receptor inhibitor.
[3] The method of [2], wherein the incubating in the presence of a TGFβ type I receptor inhibitor is incubating in a culture medium comprising the TGFβ type I receptor inhibitor, and the content of the TGFβ type I receptor inhibitor in the culture medium is 1 µM or more and 10 µM or less.
[4] The method of any one of [1] to [3], wherein the cells comprising at least fat-derived stem cells include no mature adipocyte.
[5] The method of any one of [1] to [4], wherein the fat-derived stem cells are bovine-derived.
[6] The method of any one of [1] to [5], wherein the incubating is performed for 96 hours or more and 384 hours or less.
[7] The method of any one of [1] to [6], comprising incubating the cells together with a fragmented extracellular matrix component in the presence of a TGFβ type I receptor inhibitor.
[8] The method of [7], wherein the fragmented extracellular matrix component is disposed in a gap among the cells in the three-dimensional tissue construct.
[9] The method of [7] or [8], wherein the fragmented extracellular matrix component is a fragmented collagen component.
[10] The method of any one of [7] to [9], further comprising:
   a step of bringing the cells into contact with the fragmented extracellular matrix component in an aqueous medium before incubation.
[11] A method for promoting differentiation of fat-derived stem cells, comprising incubating cells comprising at least fat-derived stem cells in the presence of one or more fatty acids selected from the group consisting of erucic acid, elaidic acid, oleic acid, palmitoleic acid, myristoleic acid, phytanic acid, and, pristanic acid.
[12] The method of [11], comprising incubating the cells in the presence of a TGFβ type I receptor inhibitor.
[13] The method of [12], wherein the incubating in the presence of a TGFβ type I receptor inhibitor is incubating in a culture medium comprising the TGFβ type I receptor inhibitor, and the content of the TGFβ type I receptor inhibitor in the culture medium is 1 µM or more and 10 µM or less.
[14] The method of any one of [11] to [13], wherein the fat-derived stem cells are bovine-derived.
[15] The method of any one of [11] to [14], wherein the incubating is performed for 96 hours or more and 384 hours or less.
[16] The method of any one of [11] to [15], comprising incubating cells comprising at least fat-derived stem cells together with a fragmented extracellular matrix component in the presence of a TGFβ type I receptor inhibitor.
[17] The method of [16], wherein the fragmented extracellular matrix component is a fragmented collagen component.
[18] The method of [16] or [17], further comprising a step of bringing the cells into contact with the fragmented extracellular matrix component in an aqueous medium before incubation.
   [P1] A method for producing a three-dimensional tissue construct comprising mature adipocytes, comprising incubating cells comprising at least fat-derived stem cells in the presence of a TGFβ type I receptor inhibitor.
   [P2] The method of [P1], comprising incubating the cells in the presence of one or more fatty acids selected from the group consisting of erucic acid, elaidic acid, oleic acid, palmitoleic acid, myristoleic acid, phytanic acid, and, pristanic acid.
   [P3] The method of [P1] or [P2], wherein the cells comprising at least fat-derived stem cells include no mature adipocyte.
   [P4] The method of any one of [P1] to [P3], wherein the incubating in the presence of a TGFβ type I receptor inhibitor is incubating in a culture medium comprising the TGFβ type I receptor inhibitor, and the content of the TGFβ type I receptor inhibitor in the culture medium is 1 µM or more and 10 µM or less.
   [P5] The method of any one of [P1] to [P4], wherein the fat-derived stem cells are bovine-derived.
   [P6] The method of any one of [P1] to [P5], wherein the incubating is performed for 96 hours or more and 384 hours or less.
   [P7] The method of any one of [P1] to [P6], comprising incubating the cells together with a fragmented extracellular matrix component in the presence of a TGFβ type I receptor inhibitor.
   [P8] The method of [P7], wherein the fragmented extracellular matrix component is disposed in a gap among the cells in the three-dimensional tissue construct.
   [P9] The method of [P7] or [P8], wherein the fragmented extracellular matrix component is a fragmented collagen component.
   [P10] The method of any one of [P7] to [P9], further comprising a step of bringing the cells into contact with the fragmented extracellular matrix component in an aqueous medium before incubation.
   [P11] A method for promoting differentiation of fat-derived stem cells, comprising incubating cells comprising at least fat-derived stem cells in the presence of a TGFβ type I receptor inhibitor.
   [P12] The method of [P11], wherein the incubating in the presence of a TGFβ type I receptor inhibitor is incubating in a culture medium comprising the TGFβ type I receptor inhibitor, and the content of the TGFβ type I receptor inhibitor in the culture medium is 1 µM or more and 10 µM or less.
   [P13] The method of [P11] or [P12], wherein the fat-derived stem cells are bovine-derived.
   [P14] The method of any one of [P11] to [P13], wherein the incubating is performed for 96 hours or more and 384 hours or less.
   [P15] The method of any one of [P11] to [P14], comprising incubating cells comprising at least fat-derived stem cells together with a fragmented extracellular matrix component in the presence of a TGFβ type I receptor inhibitor.
   [P16] The method of [P15], wherein the fragmented extracellular matrix component is a fragmented collagen component.
   [P17] The method of [P15] or [P16], further comprising a step of bringing the cells into contact with the fragmented extracellular matrix component in an aqueous medium before incubation.
   [P18] A method for promoting differentiation of fat-derived stem cells, comprising incubating cells comprising at least fat-derived stem cells in the presence of erucic acid, elaidic acid, oleic acid, palmitoleic acid, myristoleic acid, phytanic acid, and pristanic acid.
   [P19] The method of [P18], wherein the fat-derived stem cells are bovine-derived.
   [P20] The method of [P18] or [P19], wherein the incubating is performed for 96 hours or more and 384 hours or less.
   [P21] The method of any one of [P18] to [P20], comprising incubating cells comprising at least fat-derived stem cells together with a fragmented extracellular matrix component in the presence of erucic acid, elaidic acid, oleic acid, palmitoleic acid, myristoleic acid, phytanic acid, and pristanic acid.
   [P22] The method of [P21], wherein the fragmented extracellular matrix component is a fragmented collagen component.
   [P23] The method of [P21] or [P22], further comprising a step of bringing the cells into contact with the fragmented extracellular matrix component in an aqueous medium before incubation.

### Advantageous Effect of Invention

According to the present invention, the differentiation of fat-derived stem cells into mature adipocytes is promoted. Three-dimensional tissue constructs comprising mature adipocytes can be produced in a short time by promoting the differentiation into mature adipocytes.

### Brief Description of Drawings

Figure 1 is a graph representing a result of comparison of fluorescence intensity for fat staining with Nile red for three-dimensional tissue constructs produced in Production Example 1 (*p < 0.05, **p < 0.01, ***p < 0.001).
Figure 2 is a graph representing a result of comparison of fluorescence intensity for fat staining with Nile red for three-dimensional tissue constructs produced in Production Example 2 (*p < 0.05, **p < 0.01).
Figure 3 shows results of fat staining with Nile red for three-dimensional tissue constructs produced in Production Example 3. White dots indicate nuclei (Hoechst staining).
Figure 4 is a graph representing a result of comparison of increase rates of fluorescence intensity for fat staining with Nile red for three-dimensional tissue constructs produced in Production Example 3.
Figure 5 shows results of fat staining with Nile red for three-dimensional tissue constructs produced in Production Example 4. White dots indicate nuclei (Hoechst staining).
Figure 6 is a graph representing a result of comparison of increase rates of fluorescence intensity for fat staining with Nile red for three-dimensional tissue constructs produced in Production Example 4.
Figure 7 is a graph representing a result of comparison of fluorescence intensity for fat staining with Nile red for three-dimensional tissue constructs on day 3, day 7, and day 14 of differentiation in Production Example 5.
Figure 8 shows results of fat staining with Nile red for three-dimensional tissue constructs on day 3 of differentiation in Production Example 5. White dots indicate nuclei (Hoechst staining).
Figure 9 shows results of fat staining with Nile red for three-dimensional tissue constructs on day 7 of differentiation in Production Example 5. White dots indicate nuclei (Hoechst staining).
Figure 10 shows results of fat staining with Nile red for three-dimensional tissue constructs on day 14 of differentiation in Production Example 5. White dots indicate nuclei (Hoechst staining).
Figure 11 is a graph representing a result of comparison of fluorescence intensity for fat staining with Nile red for three-dimensional tissue constructs on day 7 of differentiation in Production Example 6.

### Description of Embodiments

Hereinafter, modes for implementing the present invention will be described in detail. However, the present invention is not limited to the following embodiments.

The present invention provides, as an embodiment, a method for producing a three-dimensional tissue construct comprising mature adipocytes, comprising incubating cells comprising at least fat-derived stem cells in the presence of a TGFβ type I receptor inhibitor.

Herein, the "three-dimensional tissue construct" refers to an aggregate of cells (aggregated cell population) that is artificially made by cell culture and in which cells are three-dimensionally disposed. If the three-dimensional tissue construct includes an extracellular matrix component described later, cells are three-dimensionally disposed via the extracellular matrix component. The shape of the three-dimensional tissue construct is not limited in any way, and examples thereof include sheet-like, spherical, generally spherical, ellipsoidal, generally ellipsoidal, hemispherical, generally hemispherical, semicircular, generally semicircular, cuboidal, and generally cuboidal shapes. Here, biological tissue constructs include sweat glands, lymphatic vessels, sebaceous glands, and so on, and their configurations are more complex than that of the three-dimensional tissue construct. Therefore, the three-dimensional tissue construct and biological tissues are readily distinguishable from each other.

Herein, the "cells" are not limited in any way, and may be cells derived from a mammal such as a monkey, a dog, a cat, a rabbit, a pig, a bovine, a mouse, and a rat. The site from which the cells are derived is not limited in any way, too, and the cells may be somatic cells derived, for example, from the bone, muscle, internal organ, nerve, brain, bone, skin, or blood, or germ cells. Further, the cells may be stem cells, or cultured cells such as primary cultured cells, subcultured cells, and established cells.

Herein, the "stem cells" refer to cells possessing replication competence and pluripotency. Included in stem cells are pluripotent stem cells, which possess ability to differentiate into any type of cells, and tissue stem cells (also called somatic stem cells), which possess ability to differentiate into a particular type of cells. Examples of pluripotent stem cells include embryonic stem cells (ES cells), nuclear transfer embryonic stem cells (ntES cells), and induced pluripotent stem cells (iPS cells). Examples of tissue stem cells include mesenchymal stem cells (e.g., fat-derived stem cells, bone marrow-derived stem cells), hematopoietic stem cells, and neural stem cells.

The cells at least include fat-derived stem cells. The origin of the fat-derived stem cells is not limited in any way, and stem cells collected, for example, from subcutaneous adipose tissue or epicardium-derived adipose tissue may be used. If a three-dimensional tissue construct comprising mature adipocytes, which is produced by the method of the present embodiment, is to be ultimately used to simulate tissue at a particular part in the living construct, it is preferable to use mature adipocytes derived from tissue corresponding to the tissue at the part. For the fat-derived stem cells, for example, bovine-derived, horse-derived, mouse-derived, rat-derived, pig-derived, or stem cells may be used. According to the method of the present embodiment, the differentiation into mature adipocytes can be promoted even by using bovine-derived fat-derived stem cells, which are known to be especially difficult to differentiate into mature adipocytes. Therefore, use of bovine-derived fat-derived stem cells is preferable because the advantageous effect of the present invention becomes more significant.

The cells may further include cells other than fat-derived stem cells. Examples of cells other than fat-derived stem cells include mesenchymal cells such as fibroblasts, chondrocytes, and osteoblasts, cancer cells such as large bowel cancer cells (e.g., human large bowel cancer cells (HT29)) and liver cancer cells, cardiomyocytes, epithelial cells (e.g., human gingival epithelial cells), lymphatic endothelial cells, neurons, dendritic cells, hepatocytes, adherent cells (e.g., immunocytes), smooth muscle cells (e.g., aortic smooth muscle cells (Aorta-SMCs)), pancreatic islet cells, and keratinocytes (e.g., human epidermal keratinocytes). However, it is preferable for exerting the effect of promoting the differentiation of fat-derived stem cells into mature adipocytes in the present invention that the cells comprising at least fat-derived stem cells, that is, the cells before incubation include no mature adipocyte because the advantageous effect of the present invention becomes more significant.

The three-dimensional tissue construct in the present embodiment comprises mature adipocytes. It is preferable that 90% or more of the total cell count of adipocytes in the three-dimensional tissue construct comprising mature adipocytes be mature adipocytes, and it is more preferable that all the adipocytes be mature adipocytes. Herein, the "adipocytes" refer to all types of adipocytes except fat-derived stem cells, and include mature adipocytes and adipocytes not falling within fat-derived stem cells.

The size of a lipid droplet can be used as an index indicating the degree of maturity of an adipocyte. The lipid droplet is an intracellular organelle that stores lipids such as triglyceride (neutral fat) and cholesterol, and has a droplet-like shape with the lipids covered by a monolayer of phospholipid. Expression of a protein unique to adipose tissue (e.g., perilipin) is found on the surface of the phospholipid. The size of the lipid droplet varies among adipocytes that have matured, and if the average value of the size of the lipid droplet is 20 µm or more, for example, the adipocytes can be regarded to have matured to some degree, that is, can be regarded as mature adipocytes.

The content of the adipocytes to the total cell count in the three-dimensional tissue construct may be, for example, 5% or more, 10% or more, 15% or more, 20% or more, 25% or more, or 30% or more, and may be 95% or less, 90% or less, 80% or less, or 75% or less. The content of the mature adipocytes to the total cell count in the three-dimensional tissue construct may be, for example, 5% or more, 10% or more, 15% or more, 20% or more, 25% or more, or 30% or more, and may be 95% or less, 90% or less, 80% or less, or 75% or less.

The three-dimensional tissue construct may further include cells other than mature adipocytes. Examples of the cells other than mature adipocytes include mesenchymal cells such as fibroblasts, chondrocytes, and osteoblasts, cancer cells such as large bowel cancer cells (e.g., human large bowel cancer cells (HT29)) and liver cancer cells, cardiomyocytes, epithelial cells (e.g., human gingival epithelial cells), lymphatic endothelial cells, neurons, dendritic cells, hepatocytes, adherent cells (e.g., immunocytes), smooth muscle cells (e.g., aortic smooth muscle cells (Aorta-SMCs)), pancreatic islet cells, and keratinocytes (e.g., human epidermal keratinocytes). However, it is preferable for exerting the effect of promoting the differentiation of fat-derived stem cells into mature adipocytes in the present invention that 10% or less of the total cell count in the three-dimensional tissue construct after incubation be fat-derived stem cells, it is more preferable that 5% or less of the total cell count in the three-dimensional tissue construct after incubation be fat-derived stem cells, and it is even more preferable that the three-dimensional tissue construct include no fat-derived stem cell, because the advantageous effect of the present invention becomes more significant.

The three-dimensional tissue construct may include an intercellular vascular network. If an intercellular vascular network is formed, the three-dimensional tissue construct is expected to be successfully maintained for a long period of time, and the three-dimensional tissue construct is expected to be readily engrafted in being transplanted into mammals and so on.

"Comprising an intercellular vascular network" means comprising a structure in which branched blood vessels extend among cells to surround the cells like biological tissues. Whether a vascular network like those of biological tissues is formed can be determined, for example, on the basis of the number of vascular branches and/or the vascular interbranch lengths and/or the diversity of vascular diameter in biological tissues. If the average value of the number of vascular branches in the three-dimensional tissue construct to the average value of the number of vascular branches in biological tissues is 80% or more and 150% or less, 85% or more and 130% or less, or 90% or more and 120% or less, for example, the number of vascular branches in the three-dimensional tissue construct may be determined to be similar to the number of vascular branches in biological tissues. If the average value of the number of vascular branches in the three-dimensional tissue construct is 2.5 or more and 4.5 or less or 3.0 or more and 4.2 or less, for example, the number of vascular branches in the three-dimensional tissue construct may be determined to be similar to the number of vascular branches in biological tissues. If the average value of the vascular interbranch lengths in the three-dimensional tissue construct to the average value of the vascular interbranch lengths in biological tissues is 80% or more and 150% or less, 85% or more and 130% or less, or 90% or more and 120% or less, for example, the vascular interbranch lengths in the three-dimensional tissue construct may be determined to be similar to the vascular interbranch lengths in biological tissues. In biological tissues, both thick blood vessels and thin blood vessels are observed. In view of this, if both blood vessels of large diameter (e.g., 10 µm or more and less than 25 µm) and blood vessels of small diameter (e.g., more than 0 µm and less than 10 µm) are observed like biological tissues, for example, determination may be made as having diversity of vascular diameter similar to that in biological tissues. If 60% or more, 70% or more, or 80% or more of all of the vascular diameters are distributed within more than 0 µm and less than 25 µm, for example, determination may be made as having diversity of vascular diameter similar to that in biological tissues. It is preferable that the three-dimensional tissue construct including mature adipocytes include a vascular network among adipocytes. In this case, it is preferable, in addition to comprising a vascular network, that the adipocytes to be surrounded by the blood vessels be similar to those in biological tissues. If the average value of the size of the lipid droplet of the adipocytes in the three-dimensional tissue construct according to the present embodiment is 20 µm to 180 µm or 100 µm to 180 µm, for example, the three-dimensional tissue construct may be determined to include adipocytes similar to those in biological tissues. In the above comparison between biological tissues and the three-dimensional tissue construct, biological tissues and the three-dimensional tissue construct are compared under the same conditions (e.g., per certain specified volume, per certain specified area in the case of image analysis, per certain specified sample).

It is preferable that the thickness of the three-dimensional tissue construct be 10 µm or more, it is more preferable that the thickness be 100 µm or more, and it is even more preferable that the thickness be 1000 µm or more. Such a three-dimensional tissue construct has a more similar structure to those of biological tissues, thus being preferable as an alternative for an experimental animal and a material for transplantation. The upper limit of the thickness of the three-dimensional tissue construct is not limited in any way, and may be, for example, 10 mm or less, or 3 mm or less, or 2 mm or less, or 1.5 mm or less, or 1 mm or less.

Here, if the three-dimensional tissue construct is sheet-like or cuboidal, the "thickness of the three-dimensional tissue construct" refers to the distance between the edges in the direction perpendicular to the principal plane. If unevenness is present in the principal plane, the thickness refers to the distance at the thinnest portion in the principal plane.

If the three-dimensional tissue construct is spherical or generally spherical, the thickness refers to the diameter. Or, if the three-dimensional tissue construct is ellipsoidal or generally ellipsoidal, the thickness refers to the minor axis. If the three-dimensional tissue construct is generally spherical or generally ellipsoidal and unevenness is present in the surface, the thickness refers to the shortest distance among distances between two points at which a line passing through the center of gravity of the three-dimensional tissue construct and the surface intersect.

The method of the present embodiment comprises incubating cells comprising at least fat-derived stem cells in the presence of a TGFβ type I receptor inhibitor. The differentiation of fat-derived stem cells into mature adipocytes is promoted by incubating in the presence of a TGFβ type I receptor inhibitor. In the method of the present embodiment, a three-dimensional tissue construct comprising vascular cells can be eventually produced by promoting the differentiation with fat-derived stem cells included in a three-dimensional tissue construct, or by promoting the differentiation in a situation that a three-dimensional tissue construct is formed in such a manner that fat-derived stem cells are included in the tissue construct. Accordingly, incubating cells comprising at least fat-derived stem cells in the presence of a TGFβ type I receptor inhibitor may be incubating only cells in the presence of a TGFβ type I receptor inhibitor, or incubating in the presence of a TGFβ type I receptor inhibitor in a state that cells and an extracellular matrix component are mixed together, or incubating in the presence of a TGFβ type I receptor inhibitor in a state that a three-dimensional tissue construct comprising cells and an extracellular matrix component is formed.

Examples of the TGFβ type I receptor inhibitor include inhibitors for ALK5, ALK2, ALK4, and ALK7, which are TGFβR1 kinases. The TGFβ type I receptor inhibitor is not limited in any way as long as the TGFβ type I receptor inhibitor exhibits an action of inhibiting a TGFβ type I receptor. Examples of ALK5, ALK4, and ALK7 inhibitors include 4-[4-(1,3-benzodioxol-5-yl)-5-(2-pyridinyl)-1H-imidazol-2-yl]benzamid e, and examples of ALK5 inhibitors include 2-[3-(6-methyl-2-pyridinyl)-1H-pyrazol-4-yl]-1,5-naphthyridine, 2-(3-(6-methylpyridin-2-yl)-1H-pyrazol-4-yl)-1,5-naphthyridine hydrochloride, and N-(2-fluorophenyl)-4-([1,2,4]triazolo[1,5-a]pyridin-6-yl)-5-(6-methyl-2 -pyridyl)-1H-imidazole-2-methanamine.

Incubating in the presence of a TGFβ type I receptor inhibitor may be, for example, incubating (culturing) cells comprising at least fat-derived stem cells in a culture medium comprising a TGFβ type I receptor inhibitor.

The method of the present embodiment may further comprise incubating cells comprising at least fat-derived stem cells in the presence of one or more fatty acids selected from the group consisting of erucic acid, elaidic acid, oleic acid, palmitoleic acid, myristoleic acid, phytanic acid, and pristanic acid. Incubation may be performed in the presence of two or more, three or more, four or more, five or more, or six or more fatty acids selected from the group consisting of erucic acid, elaidic acid, oleic acid, palmitoleic acid, myristoleic acid, phytanic acid, and pristanic acid, and incubation may be performed in the presence of elaidic acid, oleic acid, palmitoleic acid, myristoleic acid, phytanic acid, and pristanic acid. Alternatively, incubation may be performed in the presence of erucic acid, elaidic acid, oleic acid, palmitoleic acid, myristoleic acid, phytanic acid, and pristanic acid. The differentiation of fat-derived stem cells into mature adipocytes can be more promoted by incubating in the presence of those fatty acids. Incubation in the presence of any of the fatty acids may be performed simultaneously with incubation in the presence of the TGFβ type I receptor inhibitor, or not. In the case of simultaneous incubation, for example, any of the fatty acids may be comprised in a culture medium together with the TGFβ type I receptor inhibitor. At that time, for example, a culture medium comprising any of the fatty acids and the TGFβ type I receptor inhibitor in advance may be used, the TGFβ type I receptor inhibitor may be added to a culture medium comprising any of the fatty acids, and any of the fatty acids may be added to a culture medium comprising the TGFβ type I receptor inhibitor. In the case of non-simultaneous incubation, for example, cells may be incubated in a culture medium comprising any of the fatty acids and then in a culture medium comprising the TGFβ type I receptor inhibitor, and cells may be incubated in a culture medium comprising the TGFβ type I receptor inhibitor and then in a culture medium comprising any of the fatty acids.

The culture medium is not limited in any way, and a preferred culture medium can be selected according to the type of the cells to be cultured. The culture medium may be a solid culture medium or a liquid culture medium, but it is preferable that the culture medium be a liquid culture medium. Examples of the culture medium include the culture media Eagle's MEM, DMEM, Modified Eagle Medium (MEM), Minimum Essential Medium, RPMI, and GlutaMax Medium. For the liquid culture medium, a gelatinous culture medium like fibrin gel, hydrogel, Matrigel, collagen gel, and gelatin gel may be used. Cells may be added to a gelatinous liquid culture medium, and a liquid culture medium comprising cells may be gelled. The culture medium may be a culture medium supplemented with serum or serum-free culture medium. The culture medium may be a mixed culture medium obtained by mixing two culture media.

The amount of the TGFβ type I receptor inhibitor is needed to be an amount enough for each fat-derived stem cell to come into contact with the inhibitor, and may be, for example, 2.0 × 10⁻¹¹ mol to 2.0 × 10⁻⁸ mol or 1.0 × 10⁻⁹ mol to 2.0 × 10⁻⁸ mol per 1 × 10⁶ cells of fat-derived stem cells, or be, for example, 1.0 × 10⁻¹⁰ mol to 1.0 × 10⁻⁷ mol or 5.0 × 10⁻⁹ mol to 1.0 × 10⁻⁷ mol per 5 × 10⁶ cells of fat-derived stem cells. The amount of the TGFβ type I receptor inhibitor may be, for example, 6.0 ng to 6.0 µg or 300 ng to 6.0 µg per 1 × 10⁶ cells of fat-derived stem cells, or, for example, 30 ng to 30 µg or 1500 ng to 30 µg per 5 × 10⁶ cells of fat-derived stem cells.

In the case of incubating cells comprising at least fat-derived stem cells in a culture medium comprising the TGFβ type I receptor inhibitor, the content of the TGFβ type I receptor inhibitor in the culture medium may be, for example, 0.1 µM or more and 100 µM or less, 0.5 µM or more and 50 µM or less, 1 µM or more and 10 µM or less, or 2 µM or more and 8 µM or less, or 0.5 µM or more, 0.7 µM or more, 1 µM or more, 2 µM or more, 3 µM or more, 4 µM or more, or 5 µM or more, and 20 µM or less, 15 µM or less, 12 µM or less, 10 µM or less, 8 µM or less, or 7 µM or less.

In the case of incubating cells comprising at least fat-derived stem cells in a culture medium comprising any of the fatty acids, the content of each fatty acid in the culture medium may be, for example, 0.1 µM or more and 200 µM or less, 2 µM or more and 100 µM or less, 10 µM or more and 60 µM or less, 30 µM or more and 50 µM or less, 30 µM or more and 150 µM or less, or 80 µM or more and 120 µM or less, or 1 µM or more, 5 µM or more, 10 µM or more, 20 µM or more, 30 µM or more, 40 µM or more, 50 µM or more, 60 µM or more, 70 µM or more, 80 µM or more, or 90 µM or more, and 200 µM or less, 150 µM or less, 120 µM or less, 100 µM or less, 80 µM or less, or 70 µM or less.

The amount of each fatty acid may be, for example, 2.0 × 10⁻¹¹ mol to 4.0 × 10⁻⁸ mol or 1.0 × 10⁻⁹ mol to 2.0 × 10⁻⁸ mol per 1 × 10⁶ cells of fat-derived stem cells. The weight of each fatty acid may be, for example, 6 ng to 15 µg or 250 ng to 10 µg per 1 × 10⁶ cells of fat-derived stem cells.

The cell density in the culture medium before incubation can be appropriately determined according to the shape and thickness of the intended three-dimensional tissue construct, the size of the culture vessel, and so on. For example, the cell density in the culture medium may be 1 to 10⁸ cells/mL or 10³ to 10⁷ cells/mL.

Incubation can be carried out under preferred conditions according to the type of the cells to be cultured, without any limitation. For example, the temperature in incubation may be 20°C to 40°C, or 30°C to 37°C. The pH of the culture medium may be 6 to 8, or 7.2 to 7.4. The time period of the incubation may be 24 hours or more and 336 hours or less, 72 hours or more and 336 hours or less, 96 hours or more and 384 hours or less, or 96 hours or more and 288 hours or less. The time period of the incubation after addition of the TGFβ type I receptor inhibitor and/or any of the fatty acids may be 24 hours or more and 336 hours or less, 72 hours or more and 336 hours or less, 96 hours or more and 384 hours or less, or 96 hours or more and 288 hours or less.

The culture vessel (support) to be used for culture of the cells is not limited in any way, and may be, for example, a well insert, a low attachment plate, or a plate, for example, having a U-shaped or V-shaped bottom. The cells may be cultured with adhering to a support, the cells may be cultured without adhering to a support, and the cells may be cultured with the cells separated from a support at the middle of culturing. If the cells are cultured without adhering to a support or cultured with the cells separated from a support at the middle of culturing, it is preferable to use a plate, for example, having a U-shaped or V-shaped bottom or a low attachment plate, which inhibits the cells from adhering to a support.

The method of the present embodiment may include incubating cells comprising at least fat-derived stem cells together with a fragmented extracellular matrix component in the presence of the TGFβ type I receptor inhibitor. By incubating together with a fragmented extracellular matrix component, a three-dimensional tissue construct in which cells are three-dimensionally disposed via the fragmented extracellular matrix component can be obtained. It is preferable that the three-dimensional tissue construct be such that the fragmented extracellular matrix component is disposed in a gap among the cells. Cells in the cells may be the same type of cells, or different types of cells. In the case of using a culture medium comprising the TGFβ type I receptor inhibitor, the fragmented extracellular matrix component may be added to the culture medium simultaneously with the TGFβ type I receptor inhibitor, or added to the culture medium prior to adding the TGFβ type I receptor inhibitor, or added to the culture medium after adding the TGFβ type I receptor inhibitor.

The fragmented extracellular matrix component can be obtained by fragmenting an extracellular matrix component. Herein, the "extracellular matrix component" is an aggregate of extracellular matrix molecules formed of a plurality of extracellular matrix molecules. The extracellular matrix refers to a substance present outside of cells in organisms. Any substance can be used for the extracellular matrix as long as the substance does not adversely affect the growth of cells and the formation of a cell aggregate. Specific examples thereof include, but are not limited to, collagen, elastin, proteoglycan, fibronectin, hyaluronic acid, laminin, vitronectin, tenascin, entactin, and fibrillin. One of these may be used singly and these may be used in combination as the extracellular matrix component. The extracellular matrix component may contain, for example, a collagen component, or be a collagen component. It is preferable for the extracellular matrix component in the present embodiment to be a substance present outside of animal cells, that is, an animal extracellular matrix component.

The extracellular matrix molecule may be a modified product or variant of the above-mentioned extracellular matrix molecule as long as the extracellular matrix molecule does not adversely affect the growth of cells and the formation of a cell aggregate, and may be a polypeptide such as a chemically synthesized peptide. The extracellular matrix molecule may be an extracellular matrix molecule having repeated sequences each represented by Gly-X-Y, which are characteristic to collagen. Here, Gly represents a glycine residue, and X and Y each independently represent an arbitrary amino acid residue. A plurality of Gly-X-Y may be the same or different. Because constraints on the disposition of molecular chains are reduced by inclusion of repeated sequences each represented by Gly-X-Y, much better functions, for example, as a scaffold material in cell culture are provided. The proportion of the sequence represented by Gly-X-Y in the extracellular matrix molecule having repeated sequences each represented by Gly-X-Y may be 80% or more and is preferably 95% or more based on the total amino acid sequence. The extracellular matrix molecule may be a polypeptide having an RGD sequence. The RGD sequence refers to a sequence represented by Arg-Gly-Asp (arginine residue-glycine residue-aspartic acid residue). Because cell adhesion is much more promoted by inclusion of an RGD sequence, much better functions, for example, as a scaffold material in cell culture are provided. Examples of extracellular matrix molecules containing a sequence represented by Gly-X-Y and an RGD sequence include collagen, fibronectin, vitronectin, laminin, and cadherin.

Examples of collagen include fibrous collagen and nonfibrous collagen. The fibrous collagen refers to collagen to serve as a main component of collagen fibers, and specific examples thereof include collagen I, collagen II, and collagen III. Examples of the nonfibrous collagen include collagen IV

Examples of proteoglycan include, but are not limited to, chondroitin sulfate proteoglycan, heparan sulfate proteoglycan, keratan sulfate proteoglycan, and dermatan sulfate proteoglycan.

The extracellular matrix component may contain at least one selected from the group consisting of collagen, laminin, and fibronectin because the advantageous effect of the present invention becomes more significant, and it is preferable that the extracellular matrix component contain collagen. The collagen is preferably fibrous collagen, and more preferably collagen I. A commercially available collagen may be used as the fibrous collagen, and specific examples thereof include porcine skin-derived collagen I manufactured by NH Foods Ltd.

The extracellular matrix component may be an animal-derived extracellular matrix component. Examples of the animal species as the origin of the extracellular matrix component include, but are not limited to, humans, pigs, and bovines. For the extracellular matrix component, a component derived from one animal species may be used, and components derived from a plurality of animal species may be used in combination.

"Fragmenting" refers to reducing the size of an aggregate of the extracellular matrix molecule. Fragmentation may be carried out under conditions that cleave bonds in the extracellular matrix molecule, or under conditions that do not cleave any bond in the extracellular matrix molecule. The molecular structure of an extracellular matrix fragmented by application of physical force is typically unchanged from that before being fragmented (the molecular structure is maintained), in contrast to the case with enzymatic treatment. The fragmented extracellular matrix component may contain a defibered extracellular matrix component, which is a component obtained by defibering the above-mentioned extracellular matrix component by application of physical force. Defibering is a mode of fragmentation, and carried out, for example, under conditions that do not cleave any bond in the extracellular matrix molecule.

The method for fragmenting the extracellular matrix component is not limited in any way. The extracellular matrix component may be defibered by application of physical force such as an ultrasonic homogenizer, a stirring homogenizer, and a high-pressure homogenizer, as the method of defibering the extracellular matrix component. If a stirring homogenizer is used, the extracellular matrix component may be directly homogenized, or homogenized in an aqueous medium such as physiological saline or an organic solvent such as ethanol. In addition, a millimeter-sized or nanometer-sized defibered extracellular matrix component can be obtained by adjusting the time, rotational frequency, and so on of homogenization. A defibered extracellular matrix component can be obtained also by defibering through repeated freezing and thawing.

The fragmented extracellular matrix component may at least partly contain a defibered extracellular matrix component. Alternatively, the fragmented extracellular matrix component may consist only of a defibered extracellular matrix component. In other words, the fragmented extracellular matrix component may be a defibered extracellular matrix component. It is preferable that the defibered extracellular matrix component contain a defibered collagen component. It is preferable that the defibered collagen component maintain the triple helix structure derived from collagen. The defibered collagen component may be a component that partially maintains the triple helix structure derived from collagen.

Examples of the shape of the fragmented extracellular matrix component include being fibrous. Being fibrous refers to a shape composed of a collagen component that is filamentous, or a shape composed of an extracellular matrix component that is filamentous and intermolecularly crosslinked. At least part of the fragmented extracellular matrix component may be fibrous. A filamentous product formed by aggregation of a plurality of filamentous extracellular matrix molecules (fibril), a filamentous product formed by additional aggregation of fibrils, and a product obtained by defibering any of them, and so on fall within the fibrous extracellular matrix component. In the fibrous extracellular matrix component, RGD sequences are conserved without being broken.

The average length of the fragmented extracellular matrix component may be 100 nm or more and 400 µm or less, or 100 nm or more and 200 µm or less. In an embodiment, the average length of the fragmented extracellular matrix component may be 5 µm or more and 400 µm or less, or 10 µm or more and 400 µm or less, or 22 µm or more and 400 µm or less, or 100 µm or more and 400 µm or less. In another embodiment, from the viewpoint of achieving much better redispersibility, the average length of the fragmented extracellular matrix component may be 100 µm or less, or 50 µm or less, or 30 µm or less, or 15 µm or less, or 10 µm or less, or 1 µm or less, and 100 nm or more. It is preferable that the average length of a large proportion of the fragmented extracellular matrix component to the entire fragmented extracellular matrix component be within the above numerical range. Specifically, it is preferable that the average length of 95% of the fragmented extracellular matrix component to the entire fragmented extracellular matrix component be within the above numerical range. It is preferable that the fragmented extracellular matrix component be a fragmented collagen component with the average diameter being within the above range, and it is more preferable that the fragmented extracellular matrix component be a defibered collagen component with the average diameter being within the above range.

The average diameter of the fragmented extracellular matrix component may be 10 nm or more and 30 µm or less, or 30 nm or more and 30 µm or less, or 50 nm or more and 30 µm or less, or 100 nm or more and 30 µm or less, or 1 µm or more and 30 µm or less, or 2 µm or more and 30 µm or less, or 3 µm or more and 30 µm or less, or 4 µm or more and 30 µm or less, or 5 µm or more and 30 µm or less. It is preferable that the fragmented extracellular matrix component be a fragmented collagen component with the average diameter being within the above range, and it is more preferable that the fragmented extracellular matrix component be a defibered collagen component with the average diameter being within the above range.

The average length and average diameter of the fragmented extracellular matrix component can be determined by individually measuring the fragmented extracellular matrix component with an optical microscope and performing image analysis. Herein, "average length" refers to the average value of length in the longitudinal direction of a sample measured, and "average diameter" refers to the average value of length in the direction orthogonal to the longitudinal direction of a sample measured.

The fragmented extracellular matrix component may contain, for example, a fragmented collagen component, and may consist of a fragmented collagen component. The "fragmented collagen component" refers to a product that is obtained by fragmenting a collagen component such as a fibrous collagen component and retains the triple helix structure. It is preferable that the average length of the fragmented collagen component be 100 nm to 200 µm, it is more preferable that the average length be 22 µm to 200 µm, and it is even more preferable that the average length be 100 µm to 200 µm. It is preferable that the average diameter of the fragmented collagen component be 50 nm to 30 µm, it is more preferable that the average diameter be 4 µm to 30 µm, and it is even more preferable that the average diameter be 20 µm to 30 µm.

At least part of the fragmented extracellular matrix component may be intermolecularly or intramolecularly crosslinked. The extracellular matrix component may be intermolecularly or intramolecularly crosslinked in the extracellular matrix molecules constituting the extracellular matrix component.

Examples of the crosslinking method include, though the method is not limited in any way, physical crosslinking by application of heat, ultraviolet rays, radiation, or the like and chemical crosslinking with a crosslinking agent or by enzymatic reaction or the like. Physical crosslinking is preferred from the viewpoint that the growth of cells is not inhibited. The crosslinking (any of physical crosslinking and chemical crosslinking) may be crosslinking via covalent bonds.

If the extracellular matrix component contains a collagen component, crosslinks may be formed between collagen molecules (triple helix structures), or between collagen fine fibers formed of collagen molecules. The crosslinking may be crosslinking by heat (thermal crosslinking). Thermal crosslinking can be carried out, for example, by performing heat treatment under reduced pressure with use of a vacuum pump. If thermal crosslinking of the collagen component is performed, the extracellular matrix component may be crosslinked through formation of a peptide bond (-NH-CO-) between an amino group of a collagen molecule and a carboxy group of the same or another collagen molecule.

Alternatively, the extracellular matrix component can be crosslinked by using a crosslinking agent. The crosslinking agent may be, for example, a crosslinking agent capable of crosslinking a carboxyl group and an amino group, or a crosslinking agent capable of crosslinking amino groups. For example, aldehyde-based, carbodiimide-based, epoxide-based, and imidazole-based crosslinking agents are preferred as the crosslinking agent from the viewpoint of economic efficiency, safety, and operability, and specific examples thereof include glutaraldehyde and water-soluble carbodiimides such as 1-ethyl-3-(3-dimethylaminopropyl)carbodiimide hydrochloride and 1-cyclohexyl-3-(2-morpholinyl-4-ethyl)carbodiimide sulfonate.

Quantification of the degree of crosslinking can be appropriately selected according to the type of the extracellular matrix component, the means for crosslinking, and so on. The degree of crosslinking may be 1% or more, 2% or more, 4% or more, 8% or more, or 12% or more, and may be 30% or less, 20% or less, or 15% or less. With the degree of crosslinking being in the above range, the extracellular matrix molecules can be moderately dispersed, and redispersibility after dry storage is satisfactory.

If amino groups in the extracellular matrix component are used for crosslinking, the degree of crosslinking can be quantified on the basis of a TNBS (trinitrobenzenesulfonic acid) method. The degree of crosslinking determined by the TNBS method may be in the above-mentioned range. The degree of crosslinking determined by the TNBS method is the proportion of amino groups used for crosslinking among the amino groups possessed by the extracellular matrix. If the extracellular matrix component contains a collagen component, it is preferable that the degree of crosslinking as measured by the TNBS method be within the above range.

The degree of crosslinking may be calculated by quantifying carboxyl groups. In the case of a water-insoluble extracellular matrix component, for example, quantification may be made by using a TBO (toluidine blue O) method. The degree of crosslinking determined by the TBO method may be within the above-mentioned range.

The content of the extracellular matrix component in the three-dimensional tissue construct may be 0.01 to 90% by mass based on the three-dimensional tissue construct (dry weight), it is preferable that the content be 10 to 90% by mass, it is preferable that the content be 10 to 80% by mass, it is preferable that the content be 10 to 70% by mass, it is preferable that the content be 10 to 60% by mass, it is preferable that the content be 1 to 50% by mass, it is preferable that the content be 10 to 50% by mass, it is more preferable that the content be 10 to 30% by mass, and it is more preferable that the content be 20 to 30% by mass.

Here, the "extracellular matrix component in the three-dimensional tissue construct" refers to the extracellular matrix component constituting the three-dimensional tissue construct, and may be derived from an endogenous extracellular matrix component or derived from an exogenous extracellular matrix component.

The "endogenous extracellular matrix component" refers to an extracellular matrix component produced by extracellular matrix-producing cells. Examples of extracellular matrix-producing cells include the above-mentioned mesenchymal cells such as fibroblasts, chondrocytes, and osteoblasts. The endogenous extracellular matrix component may be fibrous or nonfibrous.

The "exogenous extracellular matrix component" refers to an extracellular matrix component supplied from the external. The animal species as the origin of the exogenous extracellular matrix component may be identical to or different from the animal species as the origin of the endogenous extracellular matrix component. Examples of the animal species as the origin include humans, pigs, and bovines. The exogenous extracellular matrix component may be an artificial extracellular matrix component.

If the extracellular matrix component is a collagen component, the exogenous extracellular matrix component is also referred to as an "exogenous collagen component", and the "exogenous collagen component", referring to a collagen component supplied from the external, is an aggregate of collagen molecules formed of a plurality of collagen molecules, and specific examples thereof include fibrous collagen and nonfibrous collagen. It is preferable that the exogenous collagen component be fibrous collagen. The fibrous collagen refers to a collagen component to serve as a main component of collagen fibers, and examples thereof include collagen I, collagen II, and collagen III. A commercially available collagen may be used as the fibrous collagen, and specific examples thereof include porcine skin-derived collagen I manufactured by NH Foods Ltd. Examples of exogenous nonfibrous collagen include collagen IV.

The animal species as the origin of the exogenous extracellular matrix component may be different from the animal species as the origin of the cells. If the cells include extracellular matrix-producing cells, the animal species as the origin of the exogenous extracellular matrix component may be different from the animal species as the origin of the extracellular matrix-producing cells. In other words, the exogenous extracellular matrix component may be a xenogeneic extracellular matrix component.

Specifically, if the three-dimensional tissue construct contains an endogenous extracellular matrix component and a fragmented extracellular matrix component, the content of the extracellular matrix component constituting the three-dimensional tissue construct refers to the total amount of the endogenous extracellular matrix component and the fragmented extracellular matrix component. The content of the extracellular matrix can be calculated from the volume of the three-dimensional tissue construct obtained and the mass of the three-dimensional tissue construct after being decellularized.

If the extracellular matrix component contained in the three-dimensional tissue construct is a collagen component, for example, examples of the method for quantifying the amount of the collagen component in the three-dimensional tissue construct include a method of quantifying hydroxyproline as follows. Hydrochloric acid (HCl) is mixed into a lysate obtained by lysing the three-dimensional tissue construct, incubation is performed at high temperature for a predetermined period of time and the temperature is then returned to room temperature, and the supernatant resulting from centrifugation is diluted to a predetermined concentration to prepare a sample. Hydroxyproline standard solution is treated in the same manner as for the sample, and then serially diluted to prepare standards. Each of the sample and standards is subjected to a predetermined treatment with hydroxyproline assay buffer and a detection reagent, and the absorbance at 570 nm is measured. The amount of the collagen component is calculated by comparing the absorbance of the sample and those of the standards. Alternatively, a lysate obtained by suspending the three-dimensional tissue construct directly in hydrochloric acid of high concentration and lysing the three-dimensional tissue construct is centrifuged to collect the supernatant, which may be used for quantification of the collagen component. The three-dimensional tissue construct to be lysed may be as it is after recovery from the culture solution, or subjected to drying treatment after recovery and lysed with the liquid components removed. If the three-dimensional tissue construct as it is after recovery from the culture solution is lysed for quantification of the collagen component, however, the measurement of weight of the three-dimensional tissue construct is expected to vary because of the influence of a culture medium component absorbed by the three-dimensional tissue construct and a culture medium residue caused by improper experimental techniques, and hence it is preferable from the viewpoint of stable measurement of the amount of the collagen component to the weight of or per unit weight of the construct to base on the weight after drying.

More specific examples of the method for quantifying the amount of the collagen component include the following method.

### (Preparation of Sample)

The whole of the three-dimensional tissue construct subjected to freeze-drying treatment is mixed with 6 mol/L HCl and incubated with a heat block at 95°C for 20 hours or more, and the temperature is then returned to room temperature. Centrifugation is performed at 13000 g for 10 minutes, and the supernatant of the sample solution is then collected. Dilution is appropriately performed with 6 mol/L HCl so that results in measurement described later fall within the range of a calibration curve, and 200 µL of the resultant is then diluted with 100 µL of ultrapure water to prepare a sample. Of the sample, 35 µL is used.

### (Preparation of Standards)

To a screw cap tube, 125 µL of standard solution (1200 µg/mL in acetic acid) and 125 µL of 12 mol/l HCl are added and mixed together, and incubated with a heat block at 95°C for 20 hours, and the temperature is then returned to room temperature. Centrifugation is performed at 13000 g for 10 minutes, the supernatant is then diluted with ultrapure water to prepare 300 µg/mL S1, and S1 is serially diluted to produce S2 (200 µg/mL), S3 (100 µg/mL), S4 (50 µg/mL), S5 (25 µg/mL), S6 (12.5 µg/mL), and S7 (6.25 µg/mL). Additionally, S8 (0 µg/mL), which consists only of 90 µL of 4 mol/l HCl, is prepared.

### (Assay)

The standards and the sample each in 35 µL are added to a plate (attached to a QuickZyme Total Collagen Assay kit, QuickZyme Biosciences). To each well, 75 µL of assay buffer (attached to the kit) is added. The plate is closed with a seal, and incubation is performed with shaking at room temperature for 20 minutes. The seal is removed, and 75 µL of detection reagent (reagent A:B = 30 µL:45 µL, attached to the kit) is added to each well. The plate is closed with a seal, the solutions are mixed by shaking and incubated at 60°C for 60 minutes. After sufficient cooling on ice, the seal is removed, and absorbance at 570 nm is measured. The amount of the collagen component is calculated by comparing the absorbance of the sample and those of the standards.

The collagen component occupying in the three-dimensional tissue construct may be specified by the area ratio or volume ratio. "Specifying by the area ratio or volume ratio" refers to calculating the ratio of the existence area of the collagen component to the entire of the three-dimensional tissue construct by any of visual observation, various microscopes, image analysis software, and so on after the collagen component in the three-dimensional tissue construct is made distinguishable from other tissue construct constituents, for example, with a known staining method (e.g., immunostaining using an anti-collagen antibody, or Masson's trichrome staining). In specifying by the area ratio, which cross-section or surface in the three-dimensional tissue construct is used to specify the area ratio is not limited, and if the three-dimensional tissue construct is spherical or in like shape, for example, specification may be made by a diagram of a cross-section passing through the generally central portion.

If the collagen component in the three-dimensional tissue construct is specified by the area ratio, for example, the proportion of the area is 0.01 to 99% based on the total area of the three-dimensional tissue construct, it is preferable that the proportion of the area be 1 to 99%, it is preferable that the proportion of the area be 5 to 90%, it is preferable that the proportion of the area be 7 to 90%, it is preferable that the proportion of the area be 20 to 90%, and it is more preferable that the proportion of the area be 50 to 90%. The "collagen component in the three-dimensional tissue construct" is as described above. The proportion of the area of the collagen component constituting the three-dimensional tissue construct refers to the proportion of the area of the total of the endogenous collagen component and exogenous collagen component. If the three-dimensional tissue construct obtained is stained with Masson's trichrome, for example, the proportion of the area of the collagen component can be calculated as the proportion of the area of the collagen component stained blue to the total area of a cross-section passing through the generally central portion of the three-dimensional tissue construct.

It is preferable that the remaining rate of the three-dimensional tissue construct after trypsin treatment with a trypsin concentration of 0.25% at a temperature of 37°C and pH 7.4 for a reaction time of 15 minutes be 70% or more, it is more preferable that the remaining rate be 80% or more, and it is even more preferable that the remaining rate be 90% or more. Such a three-dimensional tissue construct is less likely to undergo decomposition by the enzyme during or after culture, thus being stable. The remaining rate can be calculated, for example, from the mass of the three-dimensional tissue construct before and after the trypsin treatment.

The remaining rate of the three-dimensional tissue construct after collagenase treatment with a collagenase concentration of 0.25% at a temperature of 37°C and pH 7.4 for a reaction time of 15 minutes may be 70% or more, it is more preferable that the remaining rate be 80% or more, and it is even more preferable that the remaining rate be 90% or more. Such a three-dimensional tissue construct is less likely to undergo decomposition by the enzyme during or after culture, thus being stable.

Incubating cells comprising at least fat-derived stem cells together with a fragmented extracellular matrix component in the presence of a TGFβ type I receptor inhibitor may be, for example, incubating (culturing) the cells in a culture medium comprising the TGFβ type I receptor inhibitor and the fragmented extracellular matrix component. At that time, a culture medium comprising a fragmented extracellular matrix component and a TGFβ type I receptor inhibitor may be used in advance, a TGFβ type I receptor inhibitor may be added to a culture medium comprising a fragmented extracellular matrix component, and a fragmented extracellular matrix component may be added to a culture medium comprising a TGFβ type I receptor inhibitor.

A step of bringing the cells and the fragmented extracellular matrix component into contact in an aqueous medium may be further included before incubation. In this case, the TGFβ type I receptor inhibitor and/or any of the fatty acids described above may be included in the aqueous medium, or added before incubation, or added during incubation. The aqueous medium may be the same as or different from that in incubation. By incubating with the cells and the fragmented extracellular matrix component being in contact in an aqueous medium, a three-dimensional tissue construct in which the fragmented extracellular matrix component is disposed in a gap among the cells and the cells and the fragmented extracellular matrix component are three-dimensionally distributed throughout the three-dimensional tissue construct in a homogeneous manner can be produced. For example, the cells and the fragmented extracellular matrix component may be incubated in contact with each other in an aqueous medium and then further incubated in the presence of the TGFβ type I receptor inhibitor and/or any of the fatty acids. The time period of the incubation of the cells and the fragmented extracellular matrix component in contact with each other in an aqueous medium may be, for example, 12 hours to 72 hours.

The "aqueous medium" refers to a liquid containing water as an essential component. The aqueous medium is not limited in any way as long as the fragmented extracellular matrix component can be stably present. Examples of the aqueous medium include, but are not limited to, physiological saline such as phosphate-buffered saline (PBS) and liquid culture media such as Dulbecco's Modified Eagle Medium (DMEM), and a culture medium specialized for vascular endothelial cells (EGM2).

It is preferable that the pH of the aqueous medium be in the range that does not adversely affect the growth of cells and the formation of a cell aggregate. From the viewpoint of reducing loads to the cells when the aqueous medium is put into the cells, the pH of the aqueous medium may be, for example, 7.0 or more, and 8.0 or less. Specifically, the pH of the aqueous medium may be 7.0, 7.1, 7.2, 7.3, 7.4, 7.5, 7.6, 7.7, 7.8, 7.9, or 8.0. It is preferable that the aqueous medium have buffering capacity in the above pH range, and more preferably the aqueous medium is a liquid culture medium. The liquid culture medium is not limited in any way, and a preferred culture medium can be selected according to the type of the cells to be cultured. Examples of the culture medium include the culture media Eagle's MEM, DMEM, Modified Eagle Medium (MEM), Minimum Essential Medium, RPMI, and GlutaMax Medium. The culture medium may be a culture medium supplemented with serum or serum-free culture medium. Moreover, the liquid culture medium may be a mixed culture medium obtained by mixing two or more culture media.

When being dispersed in the aqueous medium, the fragmented extracellular matrix component readily comes into contact with the cells in the aqueous medium, and can promote the formation of a three-dimensional tissue construct. Examples of the contacting step include, but are not limited to, a method of mixing together an aqueous medium containing the fragmented extracellular matrix component and an aqueous medium containing the cells, a method of adding the cells to an aqueous medium containing the fragmented extracellular matrix component, a method of adding an aqueous medium containing the extracellular matrix component to a culture solution containing the cells, a method of adding the cells to an aqueous medium containing the extracellular matrix extracellular matrix component, and a method of adding the extracellular matrix component and the cells to an aqueous medium prepared in advance.

The concentration of the fragmented extracellular matrix component in the contacting step can be appropriately determined according to the shape and thickness of the intended three-dimensional tissue construct, the size of a culture vessel, and so on. For example, the concentration of the fragmented extracellular matrix component in the aqueous medium in the contacting step may be 0.1 to 90% by mass, or 1 to 30% by mass.

The amount of the fragmented extracellular matrix component in the contacting step may be, per 1.0 × 10⁶ cells, for example, 0.1 to 100 mg, 0.5 to 50 mg, 0.8 to 25 mg, 1.0 to 10 mg, 1.0 to 5.0 mg, 1.0 to 2.0 mg, or 1.0 to 1.8 mg, and may be 0.7 mg or more, 1.1 mg or more, 1.2 mg or more, 1.3 mg or more, or 1.4 mg or more, and may be 7.0 mg or less, 3.0 mg or less, 2.3 mg or less, 1.8 mg or less, 1.7 mg or less, 1.6 mg or less, or 1.5 mg or less.

It is preferable that the mass ratio between the fragmented extracellular matrix component and the cells (fragmented extracellular matrix component/cells) in the contacting step be 1/1 to 1000/1, it is more preferable that the mass ratio be 9/1 to 900/1, and it is even more preferable that the mass ratio be 10/1 to 500/1.

The amount of the fragmented extracellular matrix component in the contacting step may be 0.1% by weight to 10% by weight, 0.5% by weight to 8% by weight, or 1 to 5% by weight based on the total weight of the culture medium.

An embodiment of the method for producing a three-dimensional tissue construct may include adding fibrinogen and thrombin simultaneously or separately in the contacting step, or after the contacting step before incubation. When fibrinogen and thrombin are mixed together, they react and fibrin is formed. The content of fibrinogen may be, for example, 1 to 10 mg/mL, or 2 to 8 mg/mL, or 5 to 6 mg/mL based on the culture medium.

A step of precipitating both the fragmented extracellular matrix component and the cells in the aqueous medium may be further included after the contacting step before incubation. The distribution of the extracellular matrix component and the cells in the three-dimensional tissue construct becomes more homogeneous by carrying out such a step. The specific method is not limited in any way, and examples thereof include a method of performing a centrifugal operation for a culture solution containing the fragmented extracellular matrix component and the cells.

The cell density in the culture medium before the above-described incubation may be the same as the cell density in the aqueous medium in the contacting step.

The present invention provides, as an embodiment, a method for promoting differentiation of fat-derived stem cells, comprising incubating cells comprising at least fat-derived stem cells in the presence of a TGFβ type I receptor inhibitor. As described above, the differentiation of fat-derived stem cells into mature adipocytes can be promoted by incubating fat-derived stem cells in the presence of a TGFβ type I receptor inhibitor. The method of the present embodiment can be used even in tissue construct production including incubation of cells, and the tissue construct production may be by three-dimensional culture or by two-dimensional culture. The method of the present embodiment comprises promoting the differentiation with fat-derived stem cells included in a tissue construct, or promoting the differentiation in a situation that a tissue construct is formed in such a manner that fat-derived stem cells are included in the tissue construct, and thereby a tissue construct comprising mature adipocytes can be eventually produced.

The method of the present embodiment only needs that the differentiation of at least some of the fat-derived stem cells is promoted through the contact with the TGFβ type I receptor inhibitor, and hence is applicable not only to three-dimensional culture in the above-described production of a three-dimensional tissue construct, but also to two-dimensional culture.

The method of the present embodiment may be a method for promoting differentiation of fat-derived stem cells in the above-described method for producing a three-dimensional tissue construct.

For specific modes and the like of the method of the present embodiment, including the three-dimensional tissue construct, the cells, incubation, the fragmented extracellular matrix component, and the steps, the above-described specific modes and the like can be applied without limitation. For example, even in the method of the present embodiment, a step of bringing the cells and the fragmented extracellular matrix component into contact in the aqueous medium may be included before incubation.

The present invention provides, as an embodiment, a method for promoting differentiation of fat-derived stem cells, comprising incubating cells comprising at least fat-derived stem cells in the presence of one or more fatty acids selected from the group consisting of erucic acid, elaidic acid, oleic acid, palmitoleic acid, myristoleic acid, phytanic acid, and pristanic acid. In addition, the present invention provides, as an embodiment, a method for promoting differentiation of fat-derived stem cells, comprising incubating cells comprising at least fat-derived stem cells in the presence of erucic acid, elaidic acid, oleic acid, palmitoleic acid, myristoleic acid, phytanic acid, and pristanic acid. The differentiation of fat-derived stem cells into mature adipocytes can be promoted by incubating fat-derived stem cells in the presence of erucic acid, elaidic acid, oleic acid, palmitoleic acid, myristoleic acid, phytanic acid, and pristanic acid.

The method of the present embodiment may include incubating cells comprising at least fat-derived stem cells together with a fragmented extracellular matrix component in the presence of one or more fatty acids selected from the group consisting of erucic acid, elaidic acid, oleic acid, palmitoleic acid, myristoleic acid, phytanic acid, and pristanic acid, or incubating cells comprising at least fat-derived stem cells together with a fragmented extracellular matrix component in the presence of erucic acid, elaidic acid, oleic acid, palmitoleic acid, myristoleic acid, phytanic acid, and pristanic acid. By incubating together with a fragmented extracellular matrix component, a three-dimensional tissue construct in which cells are three-dimensionally disposed via the fragmented extracellular matrix component can be obtained. It is preferable that the three-dimensional tissue construct be such that the fragmented extracellular matrix component is disposed in a gap among the cells.

For specific modes and the like of the method of the present embodiment, including the three-dimensional tissue construct, the cells, incubation, the fragmented extracellular matrix component, and the steps, the above-described specific modes and the like can be applied without limitation. For example, even in the method of the present embodiment, a step of bringing the cells and the fragmented extracellular matrix component into contact in the aqueous medium may be included before incubation.

### Examples

Hereinafter, the present invention will be more specifically described on the basis of Examples. However, the present invention is not limited to Examples in the following.

By heating 100 mg of a sponge fragment of porcine skin-derived collagen I (manufactured by NH Foods Ltd.) at 200°C for 24 hours, a collagen component at least part of which was crosslinked (crosslinked collagen component) was obtained. No large change in appearance was found in collagen after the heating at 200°C. Into a 15-mL tube, 50 mg of the crosslinked collagen component was put, to which 5 mL of ultrapure water was added, and homogenization was performed by using a homogenizer (AS ONE Corporation VH-10) for 6 minutes to defiber the crosslinked collagen component.

Centrifugation was performed at 10000 rpm under 21°C for 10 minutes. The supernatant was sucked, and the collagen pellet was mixed with 5 mL of fresh ultrapure water to produce a collagen solution. An operation in which the tube containing the collagen solution with being kept on ice was ultrasonicated with a sonicator (Sonics and Materials, Inc. VC50) at 100 V for 20 seconds, the sonicator was removed, and the tube containing the collagen solution was cooled on ice for 10 seconds was repeated 100 times, and thereafter the collagen solution was filtered through a filter of 40 µm in pore size to afford a dispersion containing a defibered collagen component (CMF). The dispersion was freeze-dried by using a conventional method to afford a defibered collagen component (CMF) as a dried product. The average length (length) of CMF was 14.8 ± 8.2 µm (N = 20). In use, the fragmented collagen was dispersed in a culture medium (DMEM) containing serum to reach a concentration of 10 mg/mL before use.

Cells, reagents, a production method, and so on used for production of a three-dimensional tissue construct are as follows.

### (Cells and Collagen)

- FBS (Thermo Fisher Scientific, Gibco)
- Subcutaneous fat-derived stem cells (ADSCs, bovine-derived, collected from edible beef by a conventional method)
- Erucic acid (Sigma-Aldrich Co. LLC #E3385)
- Elaidic acid (Sigma-Aldrich Co. LLC #E4637)
- Oleic acid (Sigma-Aldrich Co. LLC #O1008)
- Palmitoleic acid (Sigma-Aldrich Co. LLC #P9417)
- Myristoleic acid (Sigma-Aldrich Co. LLC #M3525)
- Phytanic acid (Sigma-Aldrich Co. LLC #P4060, or Larodan Fine Chemicals AB 11-1600)
- Pristanic acid (Sigma-Aldrich Co. LLC #P6617, or Larodan Fine Chemicals AB 11-1500)
- Fibrinogen from bovine plasma Type I-S (Sigma-Aldrich Co. LLC #F8630)

### (Culture Media and Solutions)

- DMEM culture medium (high-glucose, NACALAI TESQUE, INC.): hereinafter, simple expression of DMEM refers to that containing 10% FBS.
- 50 mg/mL fibrinogen stock solution: 50 mg of fibrinogen was weighed in an Eppendorf tube, and 1 mL of DMEM (0% FBS, 1% antibiotic) was immediately added thereto. After the tube was shaken by hand for mixing, the tube was placed in a water bath at 37°C for 3 to 5 minutes, and the resultant was filtered through a filter of 0.2 µm in pore size and aliquoted into equal amounts in Eppendorf tubes for use.

### <Production of three-dimensional tissue constructs>

### Production Example 1

Onto a 96-well microplate (manufactured by IWAKI), 2 µL of FBS-free DMEM containing 1.2% by weight (final concentration) of the defibered collagen component obtained, 6 mg/mL (final concentration) fibrinogen, 3 U/mL (final concentration) thrombin, and 5 × 10⁶ cells/mL of bovine subcutaneous fat-derived stem cells was seeded and incubated for 15 minutes. After 15 minutes, 200 µL of DMEM culture medium was added.

**[Table 1]**

| No. | Fatty acid or agent contained in DMEM culture medium | Concentration |
|---|---|---|
| 1 | Erucic acid + elaidic acid + oleic acid + palmitoleic acid + myristoleic acid + phytanic acid + pristanic acid | Each 50 µM |
| 2 | Pristanic acid + phytanic acid | Each 50 µM |
| 3 | Erucic acid + elaidic acid + oleic acid + palmitoleic acid + myristoleic acid | Each 50 µM |
| 4 | Rosiglitazone | 20 µM |
| 5 | Pristanic acid | Each 50 µM |
| 6 | Phytanic acid | Each 50 µM |
| 7 | Erucic acid | Each 50 µM |
| 8 | Elaidic acid | Each 50 µM |
| 9 | Oleic acid | Each 50 µM |
| 10 | Palmitoleic acid | Each 50 µM |
| 11 | Myristoleic acid | Each 50 µM |
| 12 | Oleic acid + palmitoleic acid + myristoleic acid | Each 50 µM |

After 2 days, medium exchange was performed with each culture medium listed in Table 1 above, and culture was subsequently performed until day 5 (day 7 after seeding), day 9 (day 11 after seeding), and day 13 (day 15 after seeding) with exchanging the culture medium every 2 days to give three-dimensional tissue constructs. The three-dimensional tissue constructs obtained were each subjected to fat staining with Nile red, and Hoechst staining for staining nuclei. Fluorescence observation was performed by using the confocal quantitative image cytometer CQ1 (Yokogawa Electric Corporation), total fluorescence intensity for fat staining (Nile red) was normalized by nucleic (Hoechst) quantification and then normalized to that under undifferentiation conditions (assumed as 1, conditions with DMEM only) by using image analysis software (Image J, NIH) to calculate fluorescence intensity for fat staining (Nile red) relative to fluorescence intensity for nucleic staining (Hoechst) (two or three samples were used for each conditions).

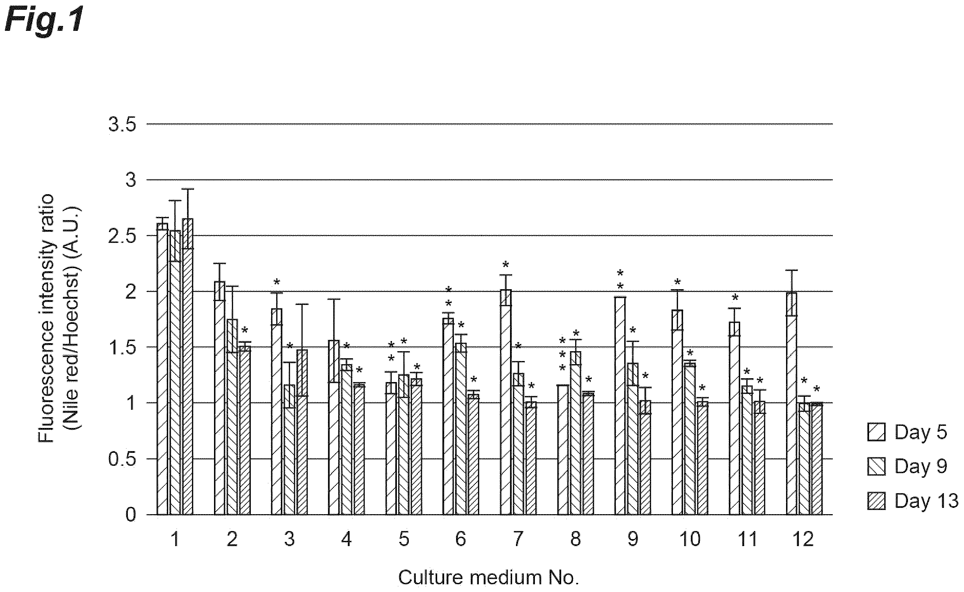

Figure 1 shows the results. In particular, it was shown that the differentiation of fat-derived stem cells was promoted in three-dimensional tissue constructs after culture for 5 days through culture in DMEM culture medium containing a free fatty acid.

### Production Example 2

Onto a 96-well plate (manufactured by IWAKI), 2 µL of FBS-free DMEM containing 1.2% by weight (final concentration) of the defibered collagen component, 6 mg/mL (final concentration) fibrinogen, 3 U/mL (final concentration) thrombin, and 5 × 10⁶ cells/mL of bovine subcutaneous fat-derived stem cells was seeded and incubated for 15 minutes, and 200 µL of a culture medium listed in Table 2 below was then added. Two days after that, medium exchange was performed with each culture medium listed in Table 2 below, and culture was performed until day 7 (day 9 after seeding) with exchanging the culture medium every 2 days to produce three-dimensional tissue constructs.

**[Table 2]**

| No. | Culture medium |
|---|---|
| 1 | DMEM + 10% FBS + insulin 10 µg/mL |
| 2 | DMEM + 10% FBS + insulin 10 µg/mL + troglitazone 40 µL |
| 3 | bovine endothelial medium + insulin 10 µg/mL |
| 4 | bovine endothelial medium + insulin 10 µg/mL + troglitazone 40 µL |
| 5 | DMEM + erucic acid, elaidic acid, oleic acid, palmitoleic acid, myristoleic acid, phytanic acid, pristanic acid |

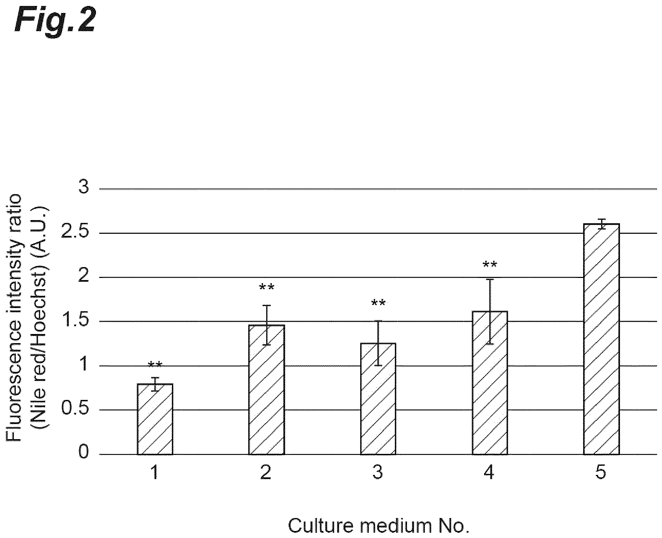

The three-dimensional tissue constructs on day 7 of differentiation (day 9 after seeding) were each subjected to Nile red staining and Hoechst staining in the same manner as in Production Example 1, and comparison was made on fluorescence intensity for fat staining (Nile red) relative to fluorescence intensity for nucleic staining (Hoechst); Figure 2 shows the result. It was shown that the differentiation of fat-derived stem cells was promoted in the three-dimensional tissue constructs cultured in the DMEM culture medium containing seven free fatty acids (No. 5) as compared with the cases with conventional cell culture media that are often used (Nos. 1 to 4).

### Production Example 3

Onto a 96-well plate (manufactured by IWAKI), 2 µL of FBS-free DMEM containing 1.2% by weight (final concentration) of the defibered collagen component, 6 mg/mL (final concentration) fibrinogen, 3 U/mL (final concentration) thrombin, and 5 × 10⁶ cells/mL of subcutaneous fat-derived stem cells was seeded and incubated for 15 minutes, 200 µL of DMEM culture medium was then added, and culture was performed for 48 hours. Thereafter, medium exchange was performed with 200 µL of DMEM culture medium containing erucic acid, elaidic acid, oleic acid, palmitoleic acid, myristoleic acid, phytanic acid, and pristanic acid each in 50 µM and an ALK5 inhibitor (2-[3-(6-methyl-2-pyridinyl)-1H-pyrazol-4-yl]-1,5-naphthyridine, Cayman Chemical Company No. 14794) in 0 µM, 1 µM, 5 µM, or 10 µM, and culture was performed with exchanging the culture medium every 2 days until day 7 of differentiation (day 9 after seeding) to give three-dimensional tissue constructs.

The three-dimensional tissue constructs obtained were each subjected to fat staining with Nile red, and Hoechst staining for staining nuclei. In the same manner as in Production Example 1, fluorescence intensity for fat staining (Nile red) relative to fluorescence intensity for nucleic staining (Hoechst) was calculated, and increase rates of fluorescence intensity with reference to that of the three-dimensional tissue constructs cultured in DMEM culture medium containing no ALK5 inhibitor were calculated (three samples were used for each conditions).

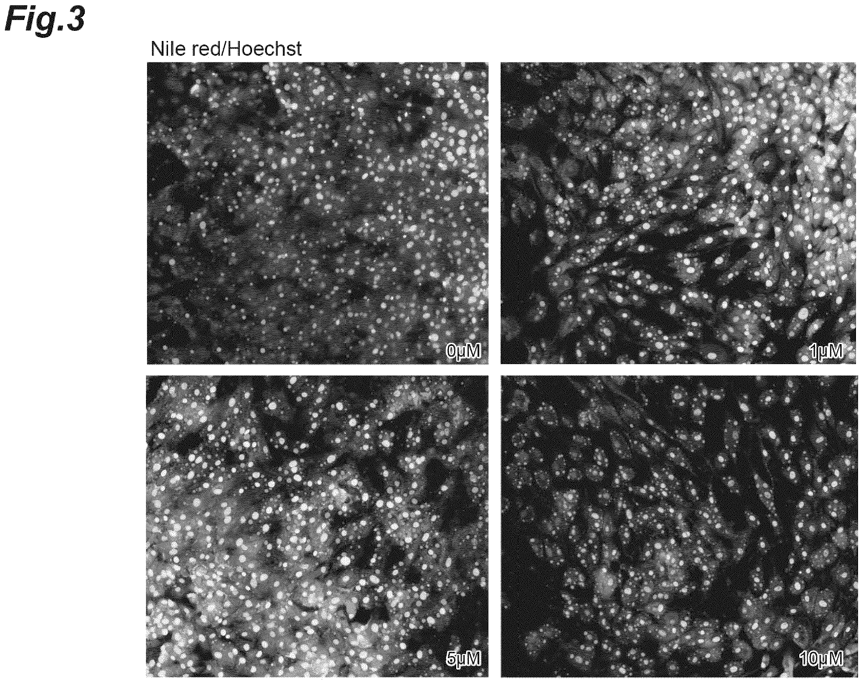

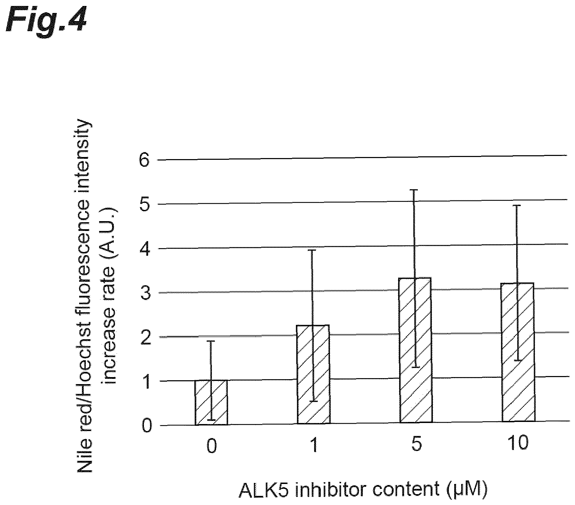

Figure 3 shows photographs of fluorescence observation for the three-dimensional tissue constructs on day 7 of differentiation (day 9 of culture) by using the confocal quantitative image cytometer CQ (Yokogawa Electric Corporation). Figure 4 shows a result of comparison of increase rates of fluorescence intensity in the three-dimensional tissue constructs.

It was found that adipogenesis had proceeded more in the three-dimensional tissue constructs each produced with DMEM culture medium containing the ALK5 inhibitor than in the three-dimensional tissue constructs produced with DMEM culture medium containing no ALK5 inhibitor (Figure 3), and the associated increase in fluorescence intensity was found (Figure 4). In particular, a 3-fold or more increase in fluorescence intensity was found for the case with DMEM culture medium containing 5 µM or more of the ALK5 inhibitor (Figure 4). It was demonstrated that the differentiation of fat-derived stem cells into mature adipocytes is promoted by addition of the ALK5 inhibitor.

### Production Example 4

Onto a 96-well plate (manufactured by IWAKI), 2 µL of FBS-free DMEM containing 1.2% by weight (final concentration) of the defibered collagen component, 6 mg/mL (final concentration) fibrinogen, 3 U/mL (final concentration) thrombin, and 5 × 10⁶ cells/mL of subcutaneous fat-derived stem cells was seeded and incubated for 15 minutes, 200 µL of DMEM culture medium was then added, and culture was performed for 48 hours. Thereafter, medium exchange was performed with 200 µL of DMEM culture medium containing erucic acid, elaidic acid, oleic acid, palmitoleic acid, myristoleic acid, phytanic acid, and pristanic acid each in 50 µM and an ALK5 inhibitor (2-[3-(6-methyl-2-pyridinyl)-1H-pyrazol-4-yl]-1,5-naphthyridine, Cayman Chemical Company No. 14794) in 5 µM, and culture was performed with exchanging the culture medium every 2 days to give three-dimensional tissue constructs.

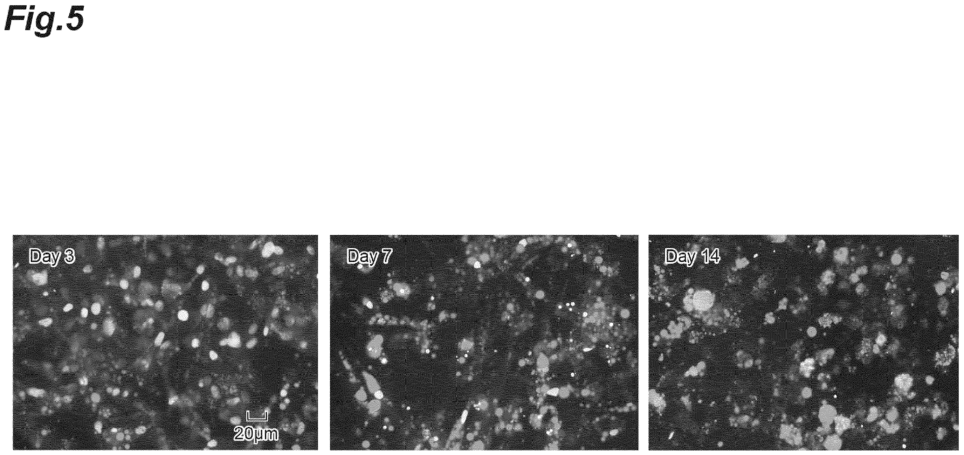

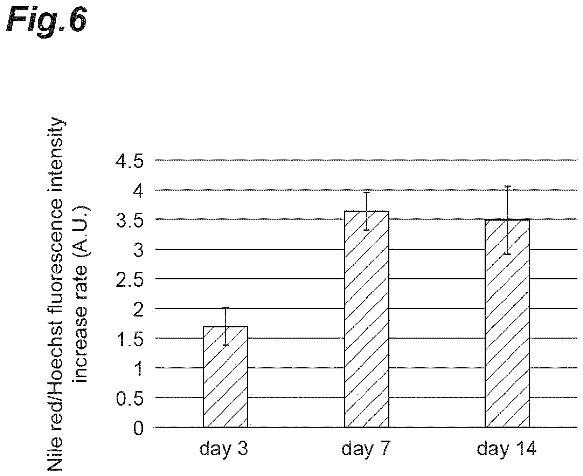

The three-dimensional tissue constructs on day 3 of differentiation (day 5 after seeding), day 7 of differentiation (day 9 after seeding), and day 14 of differentiation (day 16 after seeding) were each subjected to fat staining with Nile red, and Hoechst staining for staining nuclei; Figure 5 shows photographs of the fluorescence observation by using the confocal quantitative image cytometer CQ (Yokogawa Electric Corporation). In the same manner as in Production Example 3, those were compared with three-dimensional tissue constructs cultured in DMEM culture medium containing no ALK5 inhibitor for the same period of time; Figure 6 shows increase rates of fluorescence intensity for the three-dimensional tissue constructs.

It was found that adipogenesis had proceeded in any of the three-dimensional tissue constructs on day 3 of differentiation, day 7 of differentiation, and day 14 of differentiation (Figure 5), and the associated increase in fluorescence intensity was found (Figure 6). In particular, it was observed that adipogenesis had more proceeded in the three-dimensional tissue constructs on day 7 of culture and day 14 of culture (Figure 5), and the increase in fluorescence intensity was also significant (Figure 6). No large difference in increase rates of fluorescence intensity was found between the three-dimensional tissue constructs on day 7 of culture and those on day 14 of culture (Figure 6).

### Production Example 5

Onto a 96-well plate, 2 µL of FBS-free DMEM containing 1.2% by weight (final concentration) of the defibered collagen component, 6 mg/mL (final concentration) fibrinogen, 3 U/mL (final concentration) thrombin, and 5 × 10⁶ cells/mL of bovine subcutaneous fat-derived stem cells was seeded and incubated for 15 minutes, DMEM culture medium was then added, and culture was performed for 48 hours to give three-dimensional tissue constructs.

Culture was performed with exchanging the culture medium with 200 µL of DMEM culture medium of any of the following (1) to (3) every 2 days.
(1) DMEM culture medium containing erucic acid, elaidic acid, oleic acid, palmitoleic acid, myristoleic acid, phytanic acid, and pristanic acid each in 50 µM (1×FFA); (2) DMEM culture medium containing erucic acid, elaidic acid, oleic acid, palmitoleic acid, myristoleic acid, phytanic acid, and pristanic acid each in 100 µM (2×FFA); (3) DMEM culture medium containing erucic acid, elaidic acid, oleic acid, palmitoleic acid, myristoleic acid, phytanic acid, and pristanic acid each in 150 µM (3×FFA)

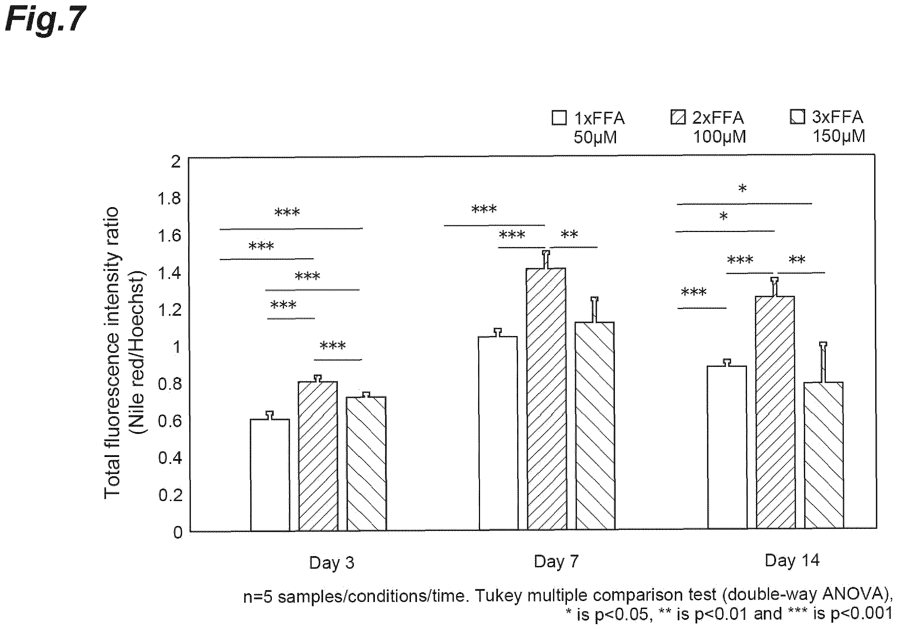

In the same manner as in Production Example 1, the three-dimensional tissue constructs in the DMEM culture media of (1) to (3) on day 3 of differentiation (day 5 after seeding), day 7 of differentiation (day 9 after seeding), and day 14 of differentiation (day 16 after seeding) were each subjected to Nile red staining and Hoechst staining, and comparison was made on fluorescence intensity for fat staining (Nile red) relative to fluorescence intensity for nucleic staining (Hoechst); Figure 7 shows the result. It was shown that the degree of maturity of adipocytes was high and thus the differentiation of fat-derived stem cells had been promoted in the three-dimensional tissue constructs cultured with DMEM culture medium containing erucic acid, elaidic acid, oleic acid, palmitoleic acid, myristoleic acid, phytanic acid, and pristanic acid, in particular, in the three-dimensional tissue constructs cultured in the culture medium containing the fatty acids each in 100 µM.

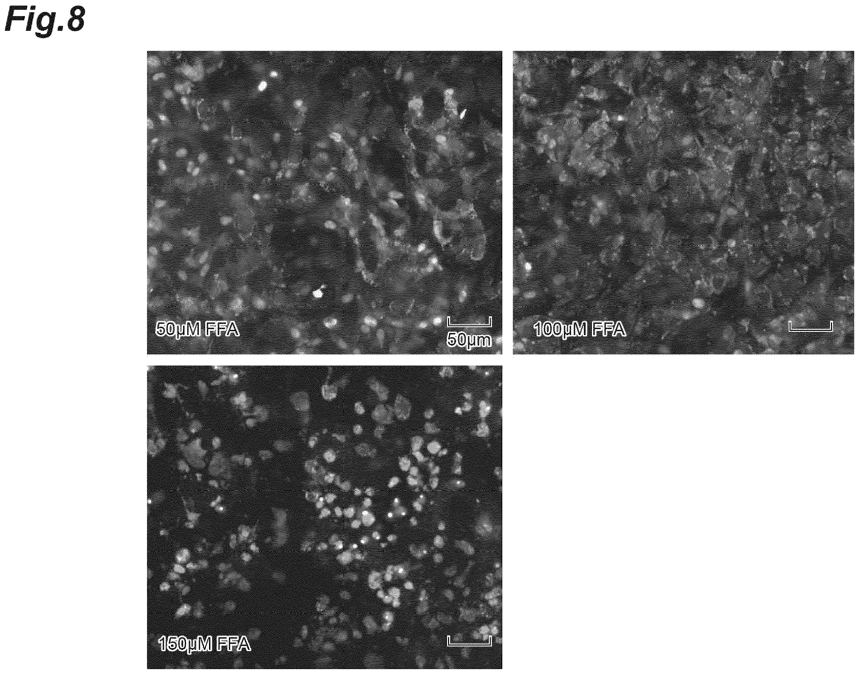

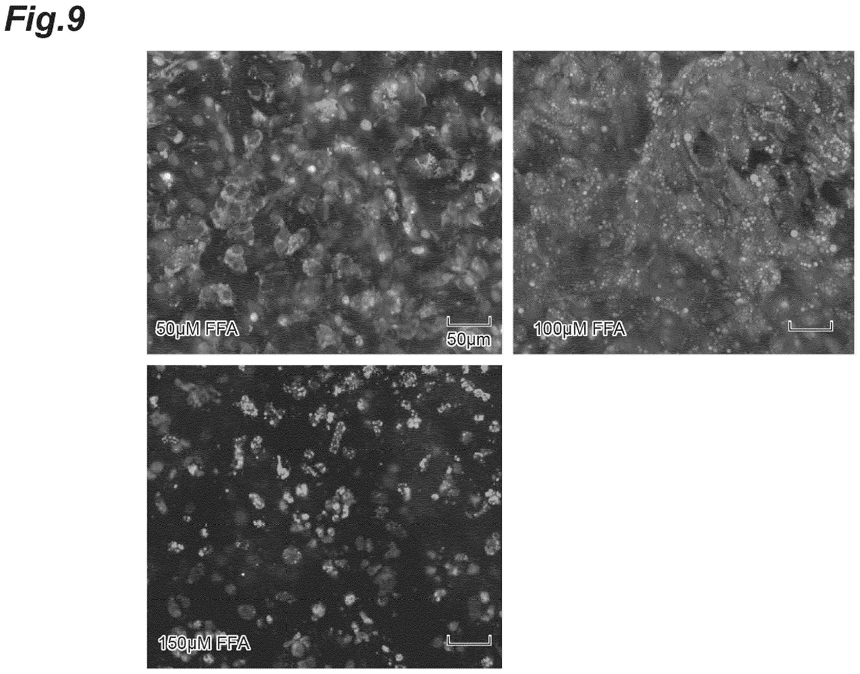

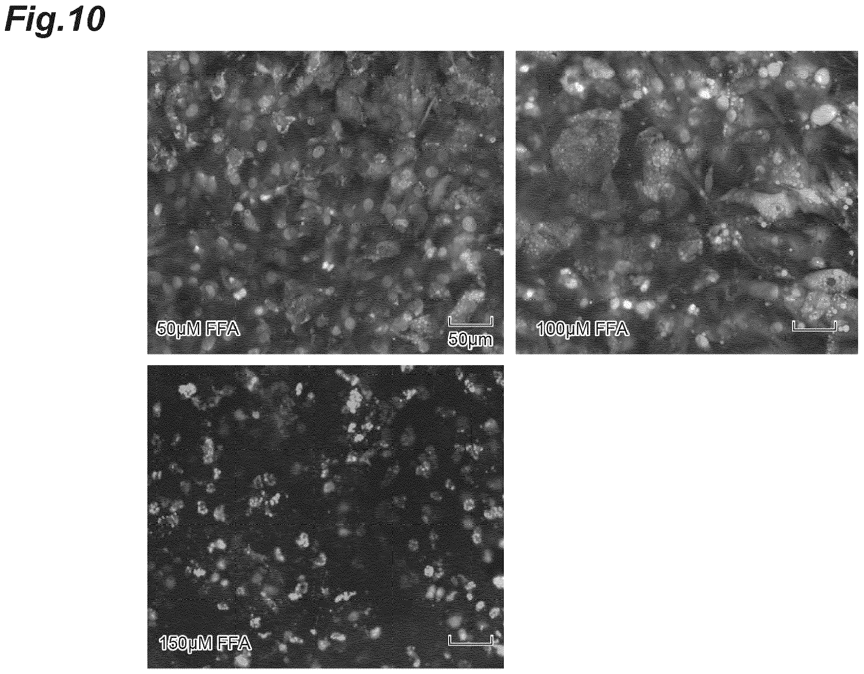

In the same manner, the three-dimensional tissue constructs on day 3 of differentiation, day 7 of differentiation, and day 14 of differentiation were subjected to Nile red staining and Hoechst staining; Figures 8 to 10 show photographs of the fluorescence observation by using the confocal quantitative image cytometer CQ (Yokogawa Electric Corporation). Figure 8 shows the three-dimensional tissue constructs on day 3 of differentiation, Figure 9 shows those on day 7 of differentiation, and Figure 10 shows those on day 14 of differentiation.

It was found that adipogenesis had proceeded in any of the three-dimensional tissue constructs on day 3 of differentiation, day 7 of differentiation, and day 14 of differentiation (Figures 8 to 10). The effect of erucic acid, elaidic acid, oleic acid, palmitoleic acid, myristoleic acid, phytanic acid, and pristanic acid to promote the differentiation of fat-derived stem cells was found even in culture after the formation of the tissue constructs.

### Production Example 6

Onto a 96-well microplate, 2 µL of FBS-free DMEM containing 1.2% by weight (final concentration) of the defibered collagen component, 6 mg/mL (final concentration) fibrinogen, 3 U/mL (final concentration) thrombin, and 5 × 10⁶ cells/mL of bovine subcutaneous fat-derived stem cells was seeded and incubated for 15 minutes, DMEM culture medium was then added, and culture was performed for 48 hours to give three-dimensional tissue constructs.

Culture was performed with exchanging the culture medium with 200 µL of DMEM culture medium of any of the following (1) and (2) every 2 days.
(1) DMEM culture medium containing erucic acid, elaidic acid, oleic acid, palmitoleic acid, myristoleic acid, phytanic acid, and pristanic acid each in 100 µM (7 FFA);
(2) DMEM culture medium containing elaidic acid, oleic acid, palmitoleic acid, myristoleic acid, phytanic acid, and pristanic acid each in 100 µM (6 FFA)

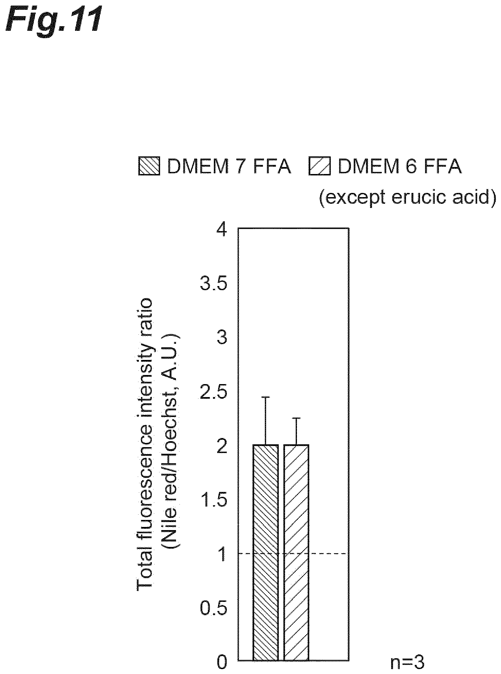

In the same manner as in Production Example 1, the three-dimensional tissue constructs in the DMEM culture media of (1) and (2) on day 7 of differentiation (day 9 after seeding) were each subjected to Nile red staining and Hoechst staining, and comparison was made on fluorescence intensity for fat staining (Nile red) relative to fluorescence intensity for nucleic staining (Hoechst); Figure 11 shows the result. It was shown that the degree of maturation of adipocytes in the three-dimensional tissue constructs cultured with the culture medium containing six fatty acids and that in the three-dimensional tissue constructs cultured with the culture medium containing seven fatty acids were comparable, and the differentiation of fat-derived stem cells had been similarly promoted.

## Claims

1. A method for producing a three-dimensional tissue construct comprising mature adipocytes, comprising incubating cells comprising at least fat-derived stem cells in the presence of one or more fatty acids selected from the group consisting of erucic acid, elaidic acid, oleic acid, palmitoleic acid, myristoleic acid, phytanic acid, and pristanic acid.

2. The method according to claim 1, comprising incubating the cells in the presence of a TGFβ type I receptor inhibitor.

3. The method according to claim 2, wherein the incubating in the presence of a TGFβ type I receptor inhibitor is incubating in a culture medium comprising the TGFβ type I receptor inhibitor, and a content of the TGFβ type I receptor inhibitor in the culture medium is 1 µM or more and 10 µM or less.

4. The method according to any one of claims 1 to 3, wherein the cells comprising at least fat-derived stem cells include no mature adipocyte.

5. The method according to any one of claims 1 to 4, wherein the fat-derived stem cells are bovine-derived.

6. The method according to any one of claims 1 to 5, wherein the incubating is performed for 96 hours or more and 384 hours or less.

7. The method according to any one of claims 1 to 6, comprising incubating the cells together with a fragmented extracellular matrix component in the presence of a TGFβ type I receptor inhibitor.

8. The method according to claim 7, wherein the fragmented extracellular matrix component is disposed in a gap among the cells in the three-dimensional tissue construct.

9. The method according to claim 7 or 8, wherein the fragmented extracellular matrix component is a fragmented collagen component.

10. The method according to any one of claims 7 to 9, further comprising a step of bringing the cells into contact with the fragmented extracellular matrix component in an aqueous medium before incubation.

11. A method for promoting differentiation of fat-derived stem cells, comprising incubating cells comprising at least fat-derived stem cells in the presence of one or more fatty acids selected from the group consisting of erucic acid, elaidic acid, oleic acid, palmitoleic acid, myristoleic acid, phytanic acid, and, pristanic acid.

12. The method according to claim 11, comprising incubating the cells in the presence of a TGFβ type I receptor inhibitor.

13. The method according to claim 12, wherein the incubating in the presence of a TGFβ type I receptor inhibitor is incubating in a culture medium comprising the TGFβ type I receptor inhibitor, and a content of the TGFβ type I receptor inhibitor in the culture medium is 1 µM or more and 10 µM or less.

14. The method according to any one of claims 11 to 13, wherein the fat-derived stem cells are bovine-derived.

15. The method according to any one of claims 11 to 14, wherein the incubating is performed for 96 hours or more and 384 hours or less.

16. The method according to any one of claims 11 to 15, comprising incubating cells comprising at least fat-derived stem cells together with a fragmented extracellular matrix component in the presence of a TGFβ type I receptor inhibitor.

17. The method according to claim 16, wherein the fragmented extracellular matrix component is a fragmented collagen component.

18. The method according to claim 16 or 17, further comprising a step of bringing the cells into contact with the fragmented extracellular matrix component in an aqueous medium before incubation.
